**Europäisches Patentamt**

(19) **European Patent Office**

**Office européen des brevets**

(11) Publication number : **0 437 518 B1**

# (12) EUROPEAN PATENT SPECIFICATION

(45) Date of publication of patent specification : **15.04.92 Bulletin 92/16**

(51) Int. Cl.⁵ : **C11C 3/08, A61K 31/23**

(21) Application number : **89911778.2**

(22) Date of filing : **10.10.89**

(86) International application number : **PCT/DK89/00239**

(87) International publication number : **WO 90/04013 19.04.90 Gazette 90/09**

(54) **TRIGLYCERIDES AND COMPOSITION COMPRISING SUCH TRIGLYCERIDES.**

(30) Priority : **10.10.88 DK 5652/88**

(43) Date of publication of application : **24.07.91 Bulletin 91/30**

(45) Publication of the grant of the patent : **15.04.92 Bulletin 92/16**

(84) Designated Contracting States : **AT BE CH DE FR GB IT LI LU NL SE**

(56) References cited :
**WO-A-88/09325**
**US-A- 4 607 052**
**US-A- 4 701 468**
**US-A- 4 701 469**

(73) Proprietor : **NOVO NORDISK A/S**
**Novo Allé**
**DK-2880 Bagsvaerd (DK)**
Proprietor : **NESTEC LTD.**
**Avenue Nestlé 55**
**CH-1800 Vevey (CH)**

(72) Inventor : **HANSEN, Tomas, Tage**
**Tonedraget 5**
**DK-3450 Alleroed (DK)**
Inventor : **GODTFREDSEN, Sven, Erik**
**Smedegade 15B**
**DK-3500 Vaerloese (DK)**
Inventor : **BRACCO, Umberto**
**Ch. Eugène Couvreu 2**
**CH-1800 Vevey (CH)**
Inventor : **WILLE, Hans-Jurgen**
**Ch. de la Colice 10**
**CH-1844 Villeneuve (CH)**

(74) Representative : **Bach, Niels et al**
**c/o Novo Nordisk A/S Patent Dept. Novo Allé**
**DK-2880 Bagsvaerd (DK)**

## Description

This invention relates to triglyceridesa and a composition comprising such triglycerides.

It appears from J. Nutr. 119:521-528, 1989 that DHA is a fatty acid which is very important as a fatty acid constituent in breast milk lipids. DHA is an abbreviation for docosahexaenoic acid, more precisely 4-7-10-13-16-19 all cis docosahexaenoic acid. In this specification with claims these three terms are considered synonyms. It appears from the article that DHA in the food, especially in relation to prematurely born children and elderly patients, is especially important for brain and retina development and, therefore, for evolvement of visual acuity and brain function.

It is a well known fact, that triglycerides containing long chain fatty acids only (e.g. DHA) are absorbed more slowly than the corresponding triglycerides containing medium chain length acids only ($C_8$-$C_{10}$) (Metabolism, Vol. 38, p. 507-513, 1989). Furthermore it is known that the human lipases, especially human pancreatic lipase, have a low activity towards polyunsaturated acids (e.g. DHA) (Lipids, Vol. 22, 711-714, 1987) and in particular towards omega-3 fatty acids. This is the case also for the lipases as e.g. the lipoprotein lipase involved in cleavage of triglycerides applied as emulsions in parenteral nutrition. Hitherto the only useful sources of DHA for enteral nutrition of the general public and e.g. prematurely born babies or for use in parenteral nutrition of e.g. the prematurely born babies have been mixtures of various DHA containing oils derived e.g. from squid, microbial triglycerides, algae or fish. Since these raw materials contain mainly long chain fatty acids they are relatively poor as energy substrates and they provide the essential fatty acids as e.g. DHA in a poor bio-available state when applied in enteral as well as in parenteral nutrition.

The purpose of the present invention is the provision of triglycerides which are devoid of the problems mentioned above regarding DHA containing triglycerides and which can thus be formulated as an enterally administrable composition, which when used as a breast milk lipid replacer or as a nutrient exhibits an improved bioavailability in regard to DHA, or which can be formulated as a parenterally administrable composition, which exhibits an improved bioavailability in regard to DHA besides being an efficient energy source.

The triglycerides according to the invention are 2-[docosahexaenoyl]-1,3-di(octanoyl/decanoyl) glycerol. It is to be understood that this term encompasses both the pure 1,3-dioctanoyl triglyceride, the pure 1,3-didecanoyl triglyceride and furthermore the 1-octanoyl-3-decanoyl triglyceride and the 1-decanoyl-3-octanoyl triglyceride, the 2-position of course in all cases being occupied by DHA. Surprisingly it has been found that these triglycerides exhibit a better bioavailability of DHA than the known DHA sources in breast milk lipid replacers and in nutrients, when formulated as an enterally administrable composition, and exhibit better bioavailability than the known sources of DHA, when formulated as a parenterally administrable composition. Also, it has been found that the enterally administrable composition according to the invention can be better tolerated and digested and exhibits a better smell and a more optimal nutrition than the previoulsy known sources of DHA. Thus, the enterally administrable composition according to the invention exhibits a vastly reduced tendency to diarrhoea, especially for infants. Moreover, the triglycerides of the invention turn out, surprisingly, to be very efficient sources of energy in enteral as well as in parenteral nutrition - the DHA moiety of the triglycerides being in an optimal position in the molecule in respect to their cleavage by lipoprotein lipase and pancreatic lipase. Also, the triglycerides of the invention appear to allow preservation of the DHA in a hydrophobic form which can pass the intestinal mucosal layer and reach the enteroside in an optimal form for further esterification, lipoprotein transport and clearance.

Surprisingly, the triglycerides of the invention appear to exhibit physical properties which allow facile formulation of the compounds in liquid products as well as in powdered products exhibiting excellent wetability properties. In the liquid form the products of the invention possess excellent stabilities making sterilization of e.g. parenteral products containing the triglycerides of the invention reliable, easy and safe.

The triglycerides of the invention allow formulation of infant feeding products with a DHA level close to that of breast milk making unnecessary use of large amounts of fish oil products or other DHA containing materials which are sensitive to oxidation and polymerisation processes. Detrimental processes of polyunsaturated fats and oils leading to negative nutritional forms are thus commonly associated with gastric and intestinal problems due to oxidation and polymerisation products of the polyunsaturated fatty acid. Such oxidized forms of DHA can interfere with nitrogen uptake by interacting with sensitive amino acids in infant formula. These highly undesired effects are diminished or even avoided by applying triglycerides according to the invention.

Application of the triglycerides of the invention in infant feeding allows red blood cell membranes, and other membranes as well, in pre-term babies to be supplemented with DHA to levels similar to those found in breast fed, normal babies. It thus appears that application of triglycerides of the invention ensures an adequate supply of DHA to blood and nerve cell membranes.

For use in parenteral feeding the triglycerides according to the invention can be incorporated into lipid emulsions where the liquid phase amounts up to 30% of the emulsion and they can be processed using the usual techniques to provide chylomicronlike particles.

The triglycerides of the invention are advantageously applied in such emulsions due to their fast conversion by lipoprotein lipase and endothelial lipase and the consequential avoidance of the discomfort and side effects of lipolipaedemia. DHA applied in triglycerides of the invention are thus cleared quickly and efficiently thereby providing the essential fatty acid concomitantly with short chain acids useful as energy substrates.

The use of triglycerides according to the invention in parenteral nutritional products is further particularly advantageous since the relatively high polarity of the triglycerides favour the stability of their emulsions which are subjected to severe heat treatments during their manufacturing. Use of such emulsion is particularly advantageous for nutrition of pre-term babies in which the desaturation and elongation system is insufficient to meet the needs.

Triglycerides of the invention can be formulated with other oils as e.g. vegetable oils. Such solutions can be used as such as a nutritional support. The triglycerides of the invention may also be formulated into creams and related products which allow the use of the compounds for topical treatment of e.g. skin diseases associated with essential fatty acid deficiencies.

This invention is a selection invention in the sense that a general chemical formula comprising the triglycerides according to the invention together with a very large number of other triglycerides belong to the prior art, vide FR 2515174, whereas the triglycerides according to the invention do not belong to the prior art. The triglycerides according to the invention are described and synthetized for the first time by the inventors, and also, the superior effect of the triglycerides according to the invention is demonstrated for the first time by the inventors.

A preferred embodiment of the triglycerides according to the invention is characterized by the fact that they have a purity of at least 5%, preferably at least 30%, more preferably at least 50%, even more preferably at least 75%, and most preferably at least 90%. The higher the purity of the triglyceride, the more efficient the absorption of the DHA in the infant or the adult.

Also, the invention comprises a nutritional composition, which comprises the triglycerides according to the invention. This composition can be either the triglycerides according to the invention without the other constituents, which are necessary for a full nutrient, i.e. mainly vitamins, proteins and carbohydrates, or the triglycerides according to the invention together with these other constituents.

A preferred embodiment of the nutritional composition according to the invention is enterally administrable. This enterally administrable composition can be used as a breast milk lipid replacer or as a nutrient, e.g. in food products like salad oil, dressing and mayonaises.

A preferred embodiment of the enteral composition according to the invention is in fluid form. The enteral composition can be an emulsion or a pure oil.

A preferred embodiment of the enteral composition according to the invention is in powder form, whereby the triglycerides are encapsulated or microencapsulated. One of the manners, in which the droplets of triglyceride can be encapsulated, is described in Danochemo Technical Information on microencapsulated Product, Malmparken 5, 2750 Ballerup, Denmark.

A preferred embodiment of the nutritional composition according to the invention is parenterally administrable. This parenterally administrable composition can be used as a nutrient.

A typical formulation including the breast milk lipid replacer according to the invention has the following composition:

Per 100 g:

| | |
|---|---|
| Fat | 25.0% |
| DHA | 0.3% |
| Protein | 11.5% |
| Carbohydrates | 58.0% |
| Minerals | 1.9% |
| Moisture | 3.0% |

diluted to provide a liquid containing 15-17% dry matter.

## EXAMPLE 1

Two columns having an internal diameter of 3.5 cm were each loaded with 30 g of immobilized lipase from *Rhizomucor miehei*, commercially available as LIPOZYME (Novo Nordisk A/S). An oil mixture consisting of 3.14 kg Japanese tuna oil (composition below) and 4.00 kg of ethyl esters of octanoic acid and decanoic acid produced by chemical ethylation of a MCT oil, were dissolved in 23.9 kg of heptane. The mixture was constantly kept under $N_2$ and at room temperature. It was then pumped through the enzyme columns, which were heated to 40°C. The flow rate was initially 64 g/h but was later adjusted down in order to keep a constant conversion.

After 121 hours of constant operation another set of enzyme column similar to the first was set up. After a continuous production for 382 hours for the first set and 239 hours for the second set the columns were stopped. The resulting mixture was evaporated under vacuum for removal of the heptane. The oil was then distilled two times on a short path distillation equipment in order to remove the ethyl esters, and then bleached and given a carbon black treatment. The final composition of the oil is given below.

Fatty acid compositions

| | Japanese tuna oil | | Product | |
|---|---|---|---|---|
| | total | 2-pos. | total | 2-pos. |
| C-8:0 | 0 | 0 | 23.9 | 4.3 |
| C-10:0 | 0 | 0 | 7.0 | 2.1 |
| C-14:0 | 2.3 | 4.4 | 1.6 | 3.3 |
| C-15:0 | 0.7 | 1.2 | 0.4 | 0.8 |
| C-16:0 | 16.5 | 24.4 | 9.2 | 17.3 |
| C-16:1 ω-7 | 4.8 | 5.7 | 2.5 | 4.0 |
| C-18:0 | 4.1 | 1.7 | 1.7 | 2.0 |
| C-18:1 ω-9 cis | 19.0 | 13.3 | 7.5 | 9.2 |
| C-18:1 ω-9 trans | 2.6 | 1.9 | 1.3 | 1.6 |
| C-18:2 ω-6 | 1.2 | 1.3 | 0.7 | 1.0 |
| C-20:1 ω-9 | 2.6 | 1.7 | 1.2 | 1.3 |
| C-20:4 ω-6 | 2.5 | 3.1 | 1.5 | 2.6 |
| C-20:5 ω-3 | 5.2 | 5.7 | 4.2 | 5.6 |
| C-22:5 ω-6 | 1.7 | 1.4 | 1.5 | 1.7 |
| C-22:5 ω-3 | 1.6 | 1.7 | 1.1 | 1.8 |
| C-22:6 ω-3 | 25.0 | 24.5 | 26.5 | 31.5 |

Only the dominating fatty acids are included in the table.

**EXAMPLE 2**

10 kg of a fish oil simular to the one in Example 1 was mixed with 40 kg of a solution of acetone/methanol in the ratio 9:1 at ÷30°C. After 2 hours at this temperature with light stirring the formed crystals were filtered off and the solvent was evaporated. The yield of DHA-enriched fish oil was 2.6 kg. The fatty acid composition was as follow:

| | |
|---|---|
| C-14:0 | 2.4 |
| C-16:0 | 9.9 |
| C-16:1 ω-7 | 5.7 |
| C-18:0 | 1.8 |
| C-18:1 ω-9, cis | 13.9 |
| C-18:1 ω-9, trans | 2.7 |
| C-18:2 ω-6 | 1.6 |
| C-18:4 ω-3 | 1.2 |
| C-20:1 ω-9 | 1.6 |
| C-20:4 ω-6 | 2.4 |
| C-20:5 ω-3 | 10.7 |
| C-22:5 ω-6 | 1.5 |

C-22:5 ω-3          2.1
C-22:6 ω-3          30.8

## EXAMPLE 3

The fractionated oil described in Example 2 was interesterified using the same method as described in Example 1. The substrate composition was: 7.85 kg of fractionated oil and 10.0 kg ethyl esters of octanoic and decanoic acid dissolved in 59.75 kg heptane. 9 enzyme columns were used, containing a total of 360 g LIPOZYME. The final composition of the oil is given below.

<u>Fatty acid composition of interesterifed product</u> (only dominating peaks)

C-8:0              15.0
C-10:0             13.7
C-14:0             1.6
C-16:0             5.4
C-16:1 ω-7         3.3
C-18:0             0.7
C-18:1 ω-9, cis    6.5
C-18:1 ω-9, trans  1.0
C-18:2 ω-6         0.7
C-18:3 ω-3         1.0
C-20:1 ω-9         0.8
C-20:4 ω-6         1.9
C-20:5 ω-3         6.9
C-22:5 ω-6         1.7
C-22:5 ω-3         1.7
C-22:6 ω-3         32.0

## Claims

1. The triglycerides 2-[docosahexaenoyl]-1,3-di(octanoyl/decanoyl) glycerol.

2. Triglycerides according to Claim 1, which have a purity of at least 5%, preferably at least 30%, more preferably at least 50%, even more preferably at least 75%, and most preferably at least 90%.

3. Nutritional composition, which comprises the triglycerides according to Claim 1 or 2.

4. Nutritional composition according to Claim 3, which is enterally administrable.

5. Nutritional composition according to Claim 4, which is in fluid form.

6. Nutritional composition according to Claim 4, which is in powder form, whereby the triglycerides are encapsulated or microencapsulated.

7. Nutritional composition according to Claim 3, which is parenterally administrable.

## Patentansprüche

1. Die Triglyceride 2-[Docosahexaenoyl]-1,3-di-(octanoyl/decanoyl)glycerin.

2. Triglyceride nach Anspruch 1, dadurch gekennzeichnet, daß sie eine Reinheit von wenigstens 5 %, vorzugsweise wenigstens 30 %, bevorzugter wenigstens 50 %, noch bevorzugter wenigstens 75 % und am bevorzugtesten wenigstens 90 % besitzen.

3. Nahrungszusammensetzung, dadurch gekennzeichnet, daß sie die Triglyceride nach Anspruch 1 oder 2 umfaßt.

4. Nahrungszusammensetzung nach Anspruch 3, dadurch gekennzeichnet, daß sie enteral verabreichbar ist.

5. Nahrungszusammensetzung nach Anspruch 4, dadurch gekennzeichnet, daß sie in flüssiger Form vorliegt.

6. Nahrungszusammensetzung nach Anspruch 4, dadurch gekennzeichnet, daß sie in Pulverform vorliegt, wobei die Triglyceride gekapselt oder mikrogekapselt sind.

7. Nahrungszusammensetzung nach Anspruch 3, dadurch gekennzeichnet, daß sie parenteral verabreichbar ist.

**Revendications**

1. Les triglycérides 2-[docosahexaénoyl]-1,3-di(octanoyl/décanoyl]glycérol.

2. Triglycérides selon la revendication 1 qui ont une pureté d'au moins 5 %, de préférence d'au moins 30 %, mieux d'au moins 50 %, encore mieux d'au moins 75 %, et tout préférablement d'au moins 90 %.

3. Composition nutritionnelle qui comprend les triglycérides selon la revendication 1 ou 2.

4. Composition nutritionnelle selon la revendication 3 qui peut être administrée par voie entérale.

5. Composition nutritionnelle selon la revendication 4 qui est sous une forme fluide.

6. Composition nutritionnelle selon la revendication 4 qui est sous forme d'une poudre, les triglycérides étant encapsulés ou microencapsulés.

7. Composition nutritionnelle selon la revendication 3 qui peut être administrée par voie parentérale.